# EUROPEAN PATENT SPECIFICATION

(11) **EP 2 429 517 B1**
(45) Date of publication and mention of the grant of the patent: **25.11.2015**
(21) Application number: 10774450.0
(22) Date of filing: 29.04.2010
(51) Int. Cl.: A61K 31/185, A01N 37/00, A61K 31/19, A61K 31/192, A61L 15/44, A61P 31/10

(54) **COMPOSITIONS SUITABLE FOR THE TOPICAL TREATMENT OF FUNGAL INFECTIONS OF THE SKIN AND NAILS**
ZUSAMMENSETZUNGEN ZUR TOPISCHEN BEHANDLUNG VON PILZINFEKTIONEN DER HAUT UND DER NÄGEL
COMPOSITIONS APPROPRIÉES POUR LE TRAITEMENT TOPIQUE D'INFECTIONS FONGIQUES DE LA PEAU ET DES ONGLES

(30) Priority: 15.05.2009 CA 2666063; 15.05.2009 US 466615
(43) Date of publication of application: 21.03.2012
(73) Proprietor: Biocepta Corporation, Toronto, ON M4P 1E2 (CA)
(72) Inventor: MLADENOVICH, Peter, Oakville, Ontario L6J 6Y5 (CA)
(74) Representative: Habets, Winand
(86) International application number: PCT/CA2010/000685
(87) International publication number: WO 2010/130028

(56) References cited:
- WO-A1-96/11572
- WO-A1-97/15282
- WO-A2-2008/152444
- AT-B- 414 096
- US-A1- 2002 183 387
- US-A1- 2004 019 112
- US-A1- 2004 096 410

## Description

### FIELD OF THE INVENTION

The present invention relates to compositions suitable for the topical treatment of fungal infections that may develop on the skin (dermatomycoses) as well as toe and finger nails (onychomycosis). These fungal infections, also commonly known as Tinea pedis (athlete's foot), Tinea unguium (nail infections), Tinea cruris (jock itch), Tinea corporis, Tinea versicolor and Tinea candidiasis, among others, are caused by different types of fungus such as those of the gena *Trichophyton, Epidermophyton, Microsporum* and *Candida.*

### BACKGROUND OF THE INVENTION

Fungal infection of the nails is one of the most common diseases of the nail bed or plate. It is estimated that between 6 and 8 percent of the adult population is affected by such fungal infections to a varying degree. Fungal infections of the nail (onychomycosis) are often caused by dermatophytic fungi, most often by one of *Trichophyton mentagrophytes* (also known as *Trichophyton interdigitale*) and *Trichophyton rubrum,* although numerous other fungi are also known to cause such infections. Fungal infection of the skin (dermatomycoses) such as Tinea pedis (athlete's foot), Tinea cruris (jock itch) and Tinea corporis are also commonly caused by *T. rubrum* and *T. mentagrophytes,* among other fungi. *T. mentagrophytes* causes a web or vesicular infection and is generally easily treated by conventional over-the-counter (OTC) medications. *T. rubrum,* however, causes a more wide-spread infection and is significantly more difficult to treat.

A large number of pharmaceutical compositions for use in the topical treatment of skin and nail fungal infections have been described in the art. These topical applications include lotions, sprays, gels and ointments containing a variety of prescription and non-prescription active ingredients.

United States Patent 6,080,744 describes cream-based topical treatments for mycotic infections consisting of a blend containing a multitude of active ingredients including ketaconazole, nystatin, miconazole nitrate, tolnaftate, clotrimazole, undecenoic acid, zinc undecenoate, propionic acid and sodium propionate. Optionally, the compositions may include additional active ingredients such as an antibacterial agent (*e.g.,* gentamicin) or an anti-inflammatory agent (e.g., dipropionate betamethasone).

European Patent Application 1 787 652 A1 describes an antifungal composition comprising *Melaleuca alternifolia* essential oil (consisting of terpinene-4-ol and cineole), a benzoate compound (benzoic acid and its salts) and the known antifungal compound miconazole nitrate.

United States Patent 5,512,200 describes compositions having a low pH comprised of a two inorganic acids (one dissociating completely in water, the second less strong than the first) and two organic acids. Among the uses taught for these compositions are pharmaceutical agents, water treatment, topical disinfectants, and the replacement of battery fluids. Owing to the low pH of these compositions, they would be expected to be particularly irritating to the skin upon topical administration, leading to problems with patient compliance and thus effective eradication of the infection.

United States Patent 6,664,292 describes compositions for the treatment of fungal infections of the nail comprised of a lower alcohol, preferably methanol, and a lower carboxylic acid.

United States Patent 4,824,865 describes compositions containing 2-hydroxyoctanoic acid, 2-ketooctanoic acid and certain esters thereof for the treatment of various skin conditions, including *tinea pedis,* in vehicles such as aqueous or non-aqueous ethanol.

United States Patent 6,214,889 describes liquid and gel compositions for the treatment of adverse skin conditions consisting of one or more of potassium, sodium, calcium or cesium formate, preferably in concentrations of about 50% in distilled water and an optional gelling agent.

United States Patent 6,921,529 describes a topical composition containing a weak organic acid dispersed in a polymeric matrix to form a supersturated hydro-gel. Optionally, the use of other, known, antimycotic agents such as azole derivatives (such as ketaconazole, miconzaole nitrate, clotrimazole), undecylenic acid, tea tree oil (*Melaleuca alternifolia*), or salicyclic acid may be added to the hydrogel.

International Patent Application 2006/042324 describes non-water soluble, film-forming compositions which adheres to body tissues comprising alkyl and hydroxyalkyl celluloses, a polar protic solvent, an antifungal agent such as naftidine, ciclopirox or terbinafine, a glycol ether, an antipruritic agent, and one or more of a solubility enhancing, surfactant and wetting agent.

United States Patent 6,159,977 describes antifungal compositions comprising an antifungal agent such as clotrimazole, tolnaftate, nystatin or undecylenic acid in combination with dimethylsulfoxide and an antiinflammatory agent, in an anhydrous solution of polyglycol.

United States Patent 7,074,392 describes antifungal compositions comprising an antifungal agent selected from common agents such as naftidine, miconazole, clotrimazole, etc., and optionally a keratolytic agent, a humectant, water, a polymeric film-forming agent such as a hydrophobic water-insoluble polymer, and a solvent system involving at least one volatile solvent. Optionally, the compositions may also include one or more of an antibacterial, antiviral or antipsoriatic agent.

United States Patent 6,231,875 describes compositions comprising an active ingredient, an acidifier (acids with low pH such as hydrochloric acid, sulfuric acid, glycolic acid, lactic acid or acetic acid), and a volatile solvent (e.g., alcohols, acetone, ethyl acetate). Active ingredients are described as preferably being antifungal drugs such as azoles (ketoconazole, fluconazole, clotrimazole), allylamines (terbinafine or naftifine), or mixtures therefore, and may also include antibacterial agents, antipsoriatic drugs, nail growth promoters, and nutrients.

Although they are present in topical antifungal compositions described in the art, a number of agents known to exhibit an antifungal property to some degree have been found to be not safe and effective by the US Food & Drug Administration (FDA) when used as active ingredients in OTC topical antifungal preparations. These agents include alcloxa, alum, aluminum sulfate, secondary amyltricresols, basic fuchsin, benzethonium chloride, benzoic acid, benzoxiquine, boric acid, camphor, candicidin, chlorothymol, coal tar, dichlorophen, menthol, methylparaben, oxyquinoline, oxyquinoline sulfate, phenol, phenolate sodium, phenyl salicylate, propionic acid, propylparaben, resorcinol, salicylic acid, sodium borate, sodium caprylate, sodium propionate, sulfur, tannic acid, thymol, tolindate, triacetin, zinc caprylate, and zinc propionate. Currently, the only antifungal agents deemed safe and effective by the FDA for use as active ingredients in OTC topical antifungal preparations are clioquinol, haloprogin, miconazole nitrate, povidone-iodine, tolnaftate, undecylenic acid and its salts, and clotrimazole. In addition to over-the-counter medications, numerous prescription medications, such as terbinafine, are also available to treat fungal infections. While terbinafine is available in various topically applied OTC formulations, they are not indicated for use in the treatment of nail infections as is its orally administered form.

Although pharmaceutical compositions using the some of the above ingredients now viewed by the FDA as not safe and effective are known and have previously been sold as over-the-counter medicaments before the FDA decision regarding their efficacy, most notably Whitfield's Ointment (a combination of benzoic and salicylic acids in an emulsifying base), they are generally regarded as ineffective. While seen as providing some relief of symptoms, relapse of the infection is almost universal following cessation of treatment with such compositions.

While a large number of different topical preparations, both prescription and non-prescription, are available for the treatment of fungal infections, most of these preparations do not have a wide enough spectrum effectively to treat the different types of persistent fungi that infect the skin and nails, in particular, fungal infections of the nail, which are often embedded deep within the nail and therefore difficult to reach with topical treatments. Additionally, some of the existing preparations contain toxic and allergenic substances, making treatment intolerable to the subject. These combined factors can lead to a treatment period that may be significantly protracted and which may not be able completely to eradicate the infection owing either to ineffectiveness or noncompliance with the administration guidelines.

As a result, there remains a need for new, inexpensive compositions that exhibit an antifungal effect sufficient to eradicate infections rather than providing a temporary respite in which infections reoccur following the cessation of treatment.

### SUMMARY OF THE INVENTION

The present compositions are suitable as broad spectrum, topical antifungal preparations for the treatment of a variety of fungal infections that may develop on the skin and nails, or which may be present and viable on surfaces which may come in contact with skin and nails. As a result, the compositions of the present invention may be used either therapeutically to treat a pre-existing infection, or as a fungicidal disinfectant to cleanse surfaces that may harbor the fungus, thereby preventing or limiting the occurrence of infections. Compositions of the present invention are comprised of a combination of low molecular weight organic acids, or their derivatives which revert to organic acids in the presence of moisture, ideally with the acids being in salt form, dissolved in a non-volatile hygroscopic carrier.

Although many of the carboxylic acids used in the present compositions have been previously known to possess fungicidal properties to varying degrees, they have been found generally not to be effective for use as the active ingredients in over-the-counter antifungal products. However, it has now been found that when used in combination, rather than as individual agents, the combination provides a synergistic enhancement of the individual antifungal effects of each component. This synergistic effect has allowed for the preparation of antifungal preparations having a broad spectrum antifungal effect without requiring the presence of common synthetic fungicidal agents, such as miconazole, clotrimazole and terbinafine, thereby reducing the cost of the preparations as well as avoiding the need for the use of prescription medicines.

As described in further detail below, a preferred composition of the present invention comprises the combination of sodium formate, zinc propionate, calcium propionate, and sodium benzoate in the hygroscopic carrier propylene glycol, with either glycerol (for a liquid formulation) or hydroxy ethyl cellulose (for a gel formulation).

The present invention is the result of the inventor's personal experience with a fungal foot and toe infection contracted at a fitness club, a common source of such infections. The fungal foot and toe infection was diagnosed as *tinea pedis* (athlete's foot) and treatment with several commercial antifungal preparations in powder and liquid forms was attempted. While the commercial preparations, including Dr. Scholl's^{®}, used to treat the fungal infection did alleviate the condition significantly, extended treatments were not able successfully to eradicate the infection. Moreover, available treatments often led to cracking of the skin, resulting in deeper infections following the return of symptoms. As a result of the failure of the available commercial preparations, some of which were irritating and inflammatory to the surrounding tissue, to eradicate the infection, alternative treatments possessing a broader anti-fungal spectrum were sought, thus leading to the present invention.

The active ingredients useful in the present compositions are combinations of two or more low molecular weight organic acids and their

Acids for use in the present compositions are formic, propionic and benzoic.

In addition to the use of the free acids, easily hydrolyzable anhydride, mixed anhydride and ester derivatives of the acids may also be used, it being understood that such derivatives would revert to their acid form during treatment through their exposure to moisture.

To add to the hygroscopicity of the organic acids as well as to raise the pH of the otherwise acidic compositions, it is preferable to use the acids in their salt forms.

The salts of organic acids useful in the present compositions are preferably metal salts. More preferably, the metal salts are those of alkali metals (*e.g.,* lithium, sodium and potassium), alkaline earth metals (e.g., magnesium and calcium) and metals exhibiting amphoteric properties (e.g., aluminum and zinc). Particularly preferred salt forms to be used in the present compositions are sodium, potassium, calcium and zinc. Alternatively, if it is desired that the compositions be prepared with the free acids, a buffering agent, such as sodium, potassium or calcium bicarbonate or other such agent, may be used to form salts of the carboxylic acids *in situ.*

Preferred active ingredients for the compositions of the present invention are sodium formate, zinc propionate, calcium propionate and sodium benzoate.

The active ingredients of the present compositions are dissolved in a carrier comprised primarily of one or more non-volatile hygroscopic solvents. The composition is, of course, non-toxic to humans and compatible with the skin. Such solvents preferably include glycerine, diglycerol, ethylene glycol, propylene glycol, sorbitol, xylitol, maltitol, low molecular weight polyglycols, such as polyethylene glycol or polypropylene glycol with molecular weight less than 500 gmol⁻¹, among others, and their derivatives, particularly monoether and ester derivatives. Most preferred as solvents are glycerine and propylene glycol. The use of the aforementioned hygroscopic solvents rather than water and lower monoalcohols such as methanol and ethanol, which are commonly used in topical antifungal preparations, has been found to be beneficial owing to their lower rates of evaporation, thereby allowing for extended contact times with the infected area. When evaporation of the solvent occurs too readily, the active ingredients of the compositions can precipitate, forming a powder, which is easily dispersed from the treatment site, making treatment less effective.

Also usable as a solvent, preferably as a co-solvent in a lesser amount, are ester derivatives of the previously mentioned hydroxy-containing solvents, such as di- and triglycerides, wherein the acid component is one of the previously mentioned carboxylic acids.

The carrier system used for the present compositions will preferably have a low to negligible water content. Such compositions demonstrate increased potency over compositions in which the carriers have higher water content.

The combination of acids in the present compositions may be added to the carrier in any amount desired, ideally from about 0.1% to about 50% total acid content by weight of the composition, the lower limit being to provide a sufficient effect for a given combination for the intended application and the upper limit being the saturation point of a given carrier system. Preferably, the total acid content is about 5% to about 30% by weight of the composition, and more preferably the total acid content is from about 10 to about 20% by weight of the composition. Within the total acid content, the individual acid components may be distributed in any proportion desired although it is preferable that any one acid does not comprise more than about 75% of the combination. Preferably, each acid is present to the extent of about 2% to about 10% by weight of the total formulation.

If desired, the viscosity of a formulation can be altered depending upon the properties exhibited by a particular mixture of acids/salts and solvent, and the intended method of treatment. This may be accomplished through the addition of one or more thickening agents such as soluble cellulose derivatives, oligosaccharides, polysaccharides, polyethylene glycol or polypropylene glycol with molecular weight greater than 1000, or through the use of colloidal suspensions such as silicon dioxide and hydrated aluminum oxide. Such agents are particularly useful for the preparation of gels and pastes, thereby allowing for longer retention at the treatment site. Preferred thickening agents are hydroxy ethyl cellulose and Aerosil^{®} 200 (a fumed silica).

The compositions of the present invention exhibit antifungal properties and are therefore useful in the topical treatment against fungi which cause various infections of the skin and nails such as tinea unguium, tinea pedis, tinea cruris, tinea corporis, tinea versicolor and tinea candidiasis. The fungi causing these tinea infections are known commonly to include the various species of *Trichophyton, Epidermophyton* and *Microsporum,* as well as *Candida.* The compositions are particularly useful in treating nail infections, which are often caused by *T. mentagrophytes* (also known as *T. interdigitale*) and *T. rubrum.* Fungus infection of the skin such as tinea pedis, tinea cruris and Tinea corporis are also known to be commonly caused by *T. rubrum* and *T. mentagrophytes* and therefore are also preferably treated with the present compositions.

As well as being applied directly to the infected area, compositions of the invention can also be pre-applied to bandages or other absorbent material, which is then applied to the infected area. Alternatively, the treated area can be wrapped in a bandage or other protective covering.

In addition to the treatment of fungal infections of the skin and nail, the compositions of the present invention may be used as a fungicidal disinfectant to cleanse surfaces that may harbour the infecting fungus, thereby preventing or limiting the occurrence of infections. Compositions of the invention, preferably ones with low viscosity, can be used as a fungicidal disinfectant for skin and surfaces which frequently come into contact with the skin such as door knobs, shopping cart handles, public showers and changing rooms.

Thus, in accordance with the invention, methods of treatment of fungal infections of the skin and nails have been provided wherein a therapeutically effective amount of the compositions described herein is applied to the infected area. As well, the use of the compositions described herein for the topical treatment of fungal infections of the skin and nails has also been provided. Further, the use of the compositions described herein in the preparation of a medicament for the topical treatment of fungal infections of the skin and nails is also provided.

### Exemplary Formulations of the Antifungal Compositions of the Invention

Formulation of the antifungal compositions of the present invention may be prepared by any means known to the skilled person. Two exemplary, but non-limiting, formulations of the present invention, a liquid and a gel formulation, are provided below. In addition to these exemplary formulations, other topical formulations, prepared using methods known to the skilled person, may also be used for the administration of the antifungal compositions of the present invention.

### Example 1 - Liquid Formulation

A preferred liquid formulation contains the following ingredients:

| Ingredient | % by Wt. |
|---|---|
| Propylene glycol | 72 |
| Sodium benzoate | 5 |
| Calcium propionate | 5 |
| Zinc propionate | 5 |
| Sodium formate | 3 |
| Glycerol | 10 |

This formulation may be prepared by, among other methods, first warming the propylene glycol and sequentially dissolving in it, the sodium benzoate, calcium propionate, zinc propionate and sodium formate. After dissolution of each of the salts, the glycerol is added, following which the mixture is cooled to room temperature.

### Example 2 - Gel Formulation

A preferred gel formulation contains the following ingredients:

| Ingredient | % by Wt. |
|---|---|
| Propylene glycol | 80 |
| Sodium benzoate | 5 |
| Calcium propionate | 5 |
| Zinc propionate | 5 |
| Sodium formate | 3 |
| Hydroxy ethyl cellulose | 2 |

This formulation may be prepared by, among other methods, first warming the propylene glycol and sequentially dissolving in it the sodium benzoate, calcium propionate, zinc propionate and sodium formate. After dissolution of each of the salts, the hydroxy ethyl cellulose is slowly added to prevent agglomeration, following which the mixture is cooled to room temperature.

In addition to the exemplary preparations described herein, the formulations may also be prepared by first dissolving the carboxylic acids and/or their salts and derivatives in water, or other relatively low boiling aqueous or non-aqueous solvents and, following the addition of this initial mixture to the carrier system to remove all or the majority of the water or low boiling solvent through heating or desiccation of the composition. This procedure can assist in the initial dissolution of the carboxylic acid components.

### Use and Efficacy of the Antifungal Compositions of the Invention

In general, the antifungal compositions are suitable to be used to treat fungal infections on the skin and nail by direct application of the composition on the infected area using, for example, a liquid or gel formulation, such as the formulations in Examples 1 and 2. Liquid preparations are particularly beneficial for infections that spread under skin folds or in narrow crevasses, such as nail beds. Gel preparations are particularly useful for open skin and nail infections which can additionally be covered by a protective film such as a cloth, glove, bandage, or wrap-around tape.

### Application of the Antifungal Compositions to Infected Nails

The antifungal efficacy of the invention was initially demonstrated by the inventor during development of the preparation for personal treatment of an "athlete's foot" infection, which subsequently spread to the nails. The inventor and many other individuals have tested compositions of the invention, experiencing eradication of the infection without reoccurrence following the completion of treatment.

In two individuals, each with fungal infections of the nail on all nails of both feet, the nails were first debrided to remove damaged nail material. Following debridement, the liquid formulation (as described in Example 1) was applied to each nail and the surrounding skin twice daily (morning and evening). Within two weeks, both individuals perceived a marked reduction in the sensation of irritation at the nails. Treatment was continued for eight weeks at which time cultures taken from one individual tested negative for nail fungus. Following cessation of treatment, reoccurrence of the infection was not reported by either individual. No irritation or other discomfort owing to the application of the invention was reported by either subject.

In two individuals, each with a fungal infection of the nail on a big toe, the liquid formulation (as described in Example 1) was applied twice daily (morning and evening) for eight weeks at which there was no evidence of infection. Prior to treatment the nails were not debrided. Following cessation of treatment, reoccurrence of the infection was not reported by either individual. No irritation or other discomfort owing to the application of the invention was reported by either subject. One of the two individuals was also suffering from a fungal infection of the skin on the top and underside of the foot and between the toes. The gel formulation (as described in Example 1) was applied to this area twice daily. Although treatment was continued for eight weeks, in conjunction with treatment of the infected nail, the redness and irritation had cleared within two days.

One individual with a fungal infection on both feet between the toes was treated twice daily (morning and evening) with the liquid formulation (as described in Example 1) for one week. At the end of treatment the infection had been eradicated and no reoccurrence was reported. No irritation or other discomfort owing to the application of the invention during treatment was reported.

One individual with fungal infections on the under arms and the back sides of the elbows was treated with the liquid formulation (as described in Example 1) twice daily (morning and evening) for five days. Following cessation of treatment, both infections had been completely eradicated; no reoccurrence was reported. No irritation or other discomfort owing to the application of the invention during treatment was reported.

While the treatment of infected nails in the above examples relied upon an eight-week application period, this should not be taken as the minimum time required for successful eradication of nail infections. An eight-week period was selected based upon the treatment periods generally indicated for fungal nail infections. Minimum effective treatment times using the present compositions may be determined in a formalized clinical trial program designed for such an outcome.

The combination of organic acids used in the present compositions are believed to exhibit a synergistic effect based on initial attempts to treat fungal infections using an aqueous solution of calcium and sodium propionate (constituents of the known antifungal agent Mycoban^{®}, which is commonly used as a food preservative). Although this treatment led to an initial clearing of fungal infections of the skin, the infections reoccurred within two weeks following cessation of treatment. In contrast, the infection could be eradicated when using a composition of the present invention combining as few as two organic acids, in particular salts of propionic acid and benzoic acid, in propylene glycol. This increased effect was further enhanced through the addition of a third organic acid.

### Confirmation of the Antifungal Activity of the Antifungal Compositions - Liquid Broth Assay

The antifungal activity of the present compositions was confirmed through *in vitro* testing using an antifungal microdilution method used in measuring the antifungal susceptibility of filamentous fungi that cause invasive infections. Fungal colonies are grown on potato glucose agar (PGA). One colony is picked and grown in Sabouraud glucose broth (SGB) at 24 °C for 3 days in the presence of test compound. Microdilution trays are incubated at 24 °C, and are read after 5 days of culture. Turbidity in the microdilution wells is scored with the aid of a reading mirror and compared with that of the growth control. A numerical score from 0 to 4 is given to each well using the following scale: 0 = optically clear or absence of growth, 1 = slight growth (25% of growth control), 2 = prominent reduction in growth (50% of growth control), 3 = slight reduction in growth (75% of growth control), 4 = no reduction in growth.

The turbidity scores (average of 3 tests) for *T. rubrum* and *T. mentragrophytes* grown in different concentrations (or dilutions) of test compounds for 5 days was as follows:
*Trichophyton rubrum*

| | | | | | | |
|---|---|---|---|---|---|---|
| Purity (%) | 0 | 0.625 | 1.25 | 2.5 | 5 | 10 |
| Formulation of Ex. 1 | 4 | 0 | 0 | 10 | 0 | 10 |
| Carrier | 4 | 4 | 4 | 14 | 2.67 | 11 |

*Trichophyton mentagrophytes*

| | | | | | | |
|---|---|---|---|---|---|---|
| Purity (%) | 0 | 0.625 | 1.25 | 2.5 | 5 | 10 |
| Formulation of Ex. 1 | 4 | 1 | 1 | 0.67 | 0 | 0 |
| Carrier | 4 | 4 | 4 | 4 | 3 | 2 |

This testing demonstrates that while the tested formulation was active against both *T*. *mentagrophytes* and *T*. *rubrum,* it exhibited a more potent antifungal against *T.* rubrum, which is generally more difficult to treat than *T. mentagrophytes.*

As many changes can be made to the provided examples without departing from the scope of the invention, it is intended that all material herein be interpreted as illustrative of the invention and not in a limiting sense.

## Claims

1. A composition comprising a combination of propionic acid and benzoic acid, salts thereof or anhydride or ester derivatives thereof, dissolved in a non-volatile hygroscopic carrier, for use as a topical anti-fungal preparation in the prevention or treatment of fungal infections.

2. A composition for use according to claim 1 additionally comprising formic acid, a pharmaceutically acceptable salt thereof or an anhydride or ester derivative thereof.

3. A composition for use according to claim 1 or 2 wherein the acids are in the salt form.

4. A composition for use according to claims 3 wherein the salt is a metal salt.

5. A composition for use according to claim 4 wherein the metal is selected from the group consisting of sodium, potassium, calcium and zinc.

6. A composition for use according to any one of claims 1 - 5 wherein the composition comprises a combination of sodium formate, zinc propionate, calcium propionate and sodium benzoate.

7. A composition for use according to any one of claims 1 - 6 wherein the non-volatile hygroscopic carrier is selected from the group consisting of polyhydroxylated solvents, and polyglycols having molecular weights of less than 500 gram per mol.

8. A composition for use according to any one of claims 1 - 6 wherein the non-volatile hygroscopic carrier is selected from the group consisting of glycerine, diglycerol, ethylene glycol, propylene glycol, sorbitol, xylitol, maltitol, polyethylene glycol and polypropylene glycol, with molecular weight less than 500 gram per mol.

9. A composition for use according to any one of claims 1 - 6 wherein the non-volatile hygroscopic carrier is selected from the group of propylene glycol and glycerine.

10. A composition for use according to any one of claims 1 - 6 wherein the non-volatile hygroscopic carrier comprises propylene glycol and glycerine.

11. A composition for use according to any one of claims 1 - 6 wherein the non-volatile hygroscopic carrier comprises propylene glycol and hydroxyl ethyl cellulose.

12. A composition for use according to any one of claims 1 - 11 wherein the combination of acids comprises 0.1% to 50% by weight of the composition.

13. A composition for use according to any one of claims 1 - 12 wherein each of the acids is present in an amount of 2 - 10% by weight of the total composition.

14. A composition for use according to any one of claims 1 - 13 wherein any one acid does not comprise more than 75% of the combination.

15. A composition for use according to any one of claims 1 - 14 wherein the composition additionally comprises a viscosity increasing agent.

16. A composition for use according to claim 15 wherein the viscosity increasing agent is selected from the group consisting of soluble cellulose derivatives, hydroxy ethyl cellulose, oligosaccharides, polysaccharides, polyethylene glycol, polypropylene glycol with a molecular weight greater than 1000 gram per mol, fuming silica and aluminum oxide.

17. A composition for use according to claim 16 wherein the viscosity increasing agent is hydroxy ethyl cellulose or fuming silica.

18. A composition for use according to any one of claims 1 - 17 wherein the fungal infection is an infection of the skin or nail.

19. A composition for use according to any one of claims 1 - 18 wherein the fungal infection is selected from the group consisting of tinea unguium, tinea pedis, tinea cruris, tinea corporis, tinea versicolor and tinea candidiasis.

20. A composition for use according to any one of claims 1 - 16 wherein the fungal infection is caused by Trichophyton rubrum or Trichophyton interdigitale.

21. A topical anti-fungal preparation for the treatment of fungal infections comprising a combination of propionic acid, formic acid and benzoic acid, salts thereof or anhydride or ester derivatives thereof, dissolved in a non-volatile hygroscopic carrier wherein the preparation comprises said acids in an amount of 0.1 % to 50% by weight of the composition, and no single acid or salt comprises more than 75% by weight of the total acid content.

22. A preparation according to claim 21 wherein the acids are in the salt form.

23. A preparation according to claim 22 wherein the salt is a metal salt.

24. A preparation according to claim 23 wherein the metal is selected from the group consisting of sodium, potassium, calcium and zinc.

25. A preparation according to any one of claims 21 -24 wherein the preparation comprises a combination of sodium formate, zinc propionate, calcium propionate and sodium benzoate.

26. A preparation according to any one of claims 21 -25 wherein the non-volatile hygroscopic carrier is selected from the group consisting of polyhydroxylated solvents, and polyglycols having molecular weights of less than 500 gram per mol.

27. A preparation according to any one of claims 21 - 26 wherein the non-volatile hygroscopic carrier is selected from the group consisting of glycerine, diglycerol, ethylene glycol, propylene glycol, sorbitol, xylitol, maltitol, polyethylene glycol and polypropylene glycol, with molecular weight less than 500 gram per mol.

28. A preparation according to any one of claims 21 - 25 wherein the non-volatile hygroscopic carrier is selected from the group of propylene glycol and glycerine.

29. A preparation according to any one of claims 21 - 25 wherein the non-volatile hygroscopic carrier comprises propylene glycol and glycerine.

30. A preparation according to any one of claims 21 - 25 wherein the non-volatile hygroscopic carrier comprises propylene glycol and hydroxyl ethyl cellulose.

31. A preparation according to any one of claims 21 - 30 wherein each of the acids is present in an amount of 2 - 10% by weight of the total composition.

32. A preparation according to any one of claims 21 - 31 wherein the preparation additionally comprises a viscosity increasing agent.

33. A preparation according to claim 32 wherein the viscosity increasing agent is selected from the group consisting of soluble cellulose derivatives, hydroxy ethyl cellulose, oligosaccharides, polysaccharides, polyethylene glycol, polypropylene glycol with a molecular weight greater than 1000 gram per mol, fuming silica and aluminum oxide.

34. A preparation according to claim 33 wherein the viscosity increasing agent is hydroxy ethyl cellulose or fuming silica.

35. A bandage or other material to be applied to skin or nails infected by a fungus, impregnated with a preparation according to any one of claims 21 - 34.

## Patentansprüche

1. Zusammensetzung, umfassend eine Kombination von Propionsäure und Benzoesäure, Salze davon oder Anhydrid- oder Esterderivate davon, gelöst in einem nichtflüchtigen hygroskopischen Träger, für die Verwendung als topisches antifungielles Präparat bei der Vorbeugung oder Behandlung von Pilzinfektionen.

2. Zusammensetzung für die Verwendung gemäß Anspruch 1, zusätzlich umfassend Ameisensäure, ein pharmazeutisch verträgliches Salz davon oder ein Anhydrid- oder Esterderivat davon.

3. Zusammensetzung für die Verwendung gemäß Anspruch 1 oder 2, wobei die Säuren in der Salzform vorliegen.

4. Zusammensetzung für die Verwendung gemäß Anspruch 3, wobei das Salz ein Metallsalz ist.

5. Zusammensetzung für die Verwendung gemäß Anspruch 4, wobei das Metall ausgewählt ist aus der Gruppe bestehend aus Natrium, Kalium, Calcium und Zink.

6. Zusammensetzung für die Verwendung gemäß einem der Ansprüche 1-5, wobei die Zusammensetzung eine Kombination von Natriumformiat, Zinkpropionat, Calciumpropionat und Natriumbenzoat umfasst.

7. Zusammensetzung für die Verwendung gemäß einem der Ansprüche 1-6, wobei der nichtflüchtige hygroskopische Träger ausgewählt ist aus der Gruppe bestehend aus polyhydroxylierten Lösungsmitteln und Polyglycolen mit Molekulargewichten von weniger als 500 Gramm pro mol.

8. Zusammensetzung für die Verwendung gemäß einem der Ansprüche 1-6, wobei der nichtflüchtige hygroskopische Träger ausgewählt ist aus der Gruppe bestehend aus Glycerin, Diglycerol, Ethylenglycol, Propylenglycol, Sorbit, Xylit, Maltit, Polyethylenglycol und Polypropylenglycol mit einem Molekulargewicht von weniger als 500 Gramm pro mol.

9. Zusammensetzung für die Verwendung gemäß einem der Ansprüche 1-6, wobei der nichtflüchtige hygroskopische Träger ausgewählt ist aus der Gruppe von Propylenglycol und Glycerin.

10. Zusammensetzung für die Verwendung gemäß einem der Ansprüche 1-6, wobei der nichtflüchtige hygroskopische Träger Propylenglycol und Glycerin umfasst.

11. Zusammensetzung für die Verwendung gemäß einem der Ansprüche 1-6, wobei der nichtflüchtige hygroskopische Träger Propylenglycol und Hydroxyethylcellulose umfasst.

12. Zusammensetzung für die Verwendung gemäß einem der Ansprüche 1-11, wobei die Kombination von Säuren 0,1 Gew.-% bis 50 Gew.-% der Zusammensetzung umfasst.

13. Zusammensetzung für die Verwendung gemäß einem der Ansprüche 1-12, wobei jede der Säuren in einer Menge von 2-10 Gew.-% der gesamten Zusammensetzung vorhanden ist.

14. Zusammensetzung für die Verwendung gemäß einem der Ansprüche 1-13, wobei keine der Säuren mehr als 75 % der Kombination umfasst.

15. Zusammensetzung für die Verwendung gemäß einem der Ansprüche 1-14, wobei die Kombination zusätzlich ein viskositätserhöhendes Mittel umfasst.

16. Zusammensetzung für die Verwendung gemäß Anspruch 15, wobei das viskositätserhöhende Mittel ausgewählt ist aus der Gruppe bestehend aus löslichen Cellulosederivaten, Hydroxyethylcellulose, Oligosacchariden, Polysacchariden, Polyethylenglycol, Polypropylenglycol mit einem Molekulargewicht von höher als 1000 Gramm pro mol, pyrogener Kieselsäure und Aluminiumoxid.

17. Zusammensetzung für die Verwendung gemäß Anspruch 16, wobei das viskositätserhöhende Mittel Hydroxyethylcellulose oder pyrogene Kieselsäure ist.

18. Zusammensetzung für die Verwendung gemäß einem der Ansprüche 1-17, wobei die Pilzinfektion eine Infektion von Haut oder Nägeln ist.

19. Zusammensetzung für die Verwendung gemäß einem der Ansprüche 1-18, wobei die Pilzinfektion ausgewählt ist aus der Gruppe bestehend aus Tinea unguium, Tinea pedis, Tinea cruris, Tinea corporis, Tinea versicolor und Tinea candidiadis.

20. Zusammensetzung für die Verwendung gemäß einem der Ansprüche 1-16, wobei die Pilzinfektion von Trichophyton rubrum oder Trichophyton interdigitale verursacht ist.

21. Topisches antifungielles Präparat für die Behandlung von Pilzinfektionen, umfassend eine Kombination von Propionsäure, Ameisensäure und Benzoesäure, Salze davon oder Anhydrid- oder Esterderivate davon, gelöst in einem nichtflüchtigen hygroskopischen Träger, wobei das Präparat die Säuren in einer Menge von 0,1 Gew.-% bis 50 Gew.-% der Zusammensetzung umfasst und keine/kein einzelne/einzelnes Säure oder Salz mehr als 75 Gew.-% des gesamten Säuregehalts umfasst.

22. Präparat gemäß Anspruch 21, wobei die Säuren in der Salzform vorliegen.

23. Präparat gemäß Anspruch 22, wobei das Salz ein Metallsalz ist.

24. Präparat gemäß Anspruch 23, wobei das Metall ausgewählt ist aus der Gruppe bestehend aus Natrium, Kalium, Calcium und Zink.

25. Präparat gemäß einem der Ansprüche 21-24, wobei das Präparat eine Kombination von Natriumformiat, Zinkpropionat, Calciumpropionat und Natriumbenzoat umfasst.

26. Präparat gemäß einem der Ansprüche 21-25, wobei der nichtflüchtige hygroskopische Träger ausgewählt ist aus der Gruppe bestehend aus polyhydroxylierten Lösungsmitteln und Polyglycolen mit Molekulargewichten von weniger als 500 Gramm pro mol.

27. Präparat gemäß einem der Ansprüche 21-26, wobei der nichtflüchtige hygroskopische Träger ausgewählt ist aus der Gruppe bestehend aus Glycerin, Diglycerol, Ethylenglycol, Propylenglycol, Sorbit, Xylit, Maltit, Polyethylenglycol und Polypropylenglycol mit einem Molekulargewicht von weniger als 500 Gramm pro mol.

28. Präparat gemäß einem der Ansprüche 21-25, wobei der nichtflüchtige hygroskopische Träger ausgewählt ist aus der Gruppe von Propylenglycol und Glycerin.

29. Präparat gemäß einem der Ansprüche 21-25, wobei der nichtflüchtige hygroskopische Träger Propylenglycol und Glycerin umfasst.

30. Präparat gemäß einem der Ansprüche 21-25, wobei, wobei der nichtflüchtige hygroskopische Träger Propylenglycol und Hydroxyethylcellulose umfasst.

31. Präparat gemäß einem der Ansprüche 21-30, wobei jede der Säuren in einer Menge von 2-10 Gew.-% der gesamten Zusammensetzung vorhanden ist.

32. Präparat gemäß einem der Ansprüche 21-31, wobei das Präparat zusätzlich ein viskositätserhöhendes Mittel umfasst.

33. Präparat gemäß Anspruch 32, wobei das viskositätserhöhende Mittel ausgewählt ist aus der Gruppe bestehend aus löslichen Cellulosederivaten, Hydroxyethylcellulose, Oligosacchariden, Polysacchariden, Polyethylenglycol, Polypropylenglycol mit einem Molekulargewicht von höher als 1000 Gramm pro mol, pyrogener Kieselsäure und Aluminiumoxid.

34. Präparat gemäß Anspruch 33, wobei das viskositätserhöhende Mittel Hydroxyethylcellulose oder pyrogene Kieselsäure ist.

35. Verband oder anderes Material zum Aufbringen auf von einem Pilz infizierte Haut oder Nägel, imprägniert mit einem Präparat gemäß einem der Ansprüche 21-34.

## Revendications

1. Composition comprenant une combinaison d'acide propionique et d'acide benzoïque, des sels de ceux-ci ou des dérivés d'anhydride ou d'ester de ceux-ci, solubilisés dans un véhicule hygroscopique non volatil, pour une utilisation comme préparation antifongique topique dans la prévention ou le traitement d'infections fongiques.

2. Composition pour une utilisation selon la revendication 1, comprenant en outre de l'acide formique, un sel pharmaceutiquement acceptable de celui-ci ou un dérivé d'anhydride ou d'ester de celui-ci.

3. Composition pour une utilisation selon la revendication 1 ou 2, **caractérisée en ce que** les acides se trouvent sous forme de sel.

4. Composition pour une utilisation selon la revendication 3, **caractérisée en ce que** le sel est un sel métallique.

5. Composition pour une utilisation selon la revendication 4, **caractérisée en ce que** le métal est choisi dans le groupe constitué par le sodium, le potassium, le calcium et le zinc.

6. Composition pour une utilisation selon l'une quelconque des revendications 1-5, **caractérisée en ce que** la composition comprend une combinaison de formiate de sodium, de propionate de zinc, de propionate de calcium et de benzoate de sodium.

7. Composition pour une utilisation selon l'une quelconque des revendications 1-6, **caractérisée en ce que** le véhicule hygroscopique non volatil est choisi dans le groupe constitué par les solvants polyhydroxylés, et les polyglycols ayant des poids moléculaires inférieurs à 500 grammes par mol.

8. Composition pour une utilisation selon l'une quelconque des revendications 1-6, **caractérisée en ce que** le véhicule hygroscopique non volatil est choisi dans le groupe constitué par le glycérol, le diglycérol, l'éthylène glycol, le propylène glycol, le sorbitol, le xylitol, le maltitol, le polyéthylène glycol et le polypropylène glycol, ayant un poids moléculaire inférieur à 500 grammes par mol.

9. Composition pour une utilisation selon l'une quelconque des revendications 1-6, **caractérisée en ce que** le véhicule hygroscopique non volatil est choisi dans le groupe constitué par le propylène glycol et le glycérol.

10. Composition pour une utilisation selon l'une quelconque des revendications 1-6, **caractérisée en ce que** le véhicule hygroscopique non volatil comprend du propylène glycol et du glycérol.

11. Composition pour une utilisation selon l'une quelconque des revendications 1-6, **caractérisée en ce que** le véhicule hygroscopique non volatil comprend du propylène glycol et de l'hydroxyéthylcellulose.

12. Composition pour une utilisation selon l'une quelconque des revendications 1-11, **caractérisée en ce que** la combinaison d'acides constitue de 0,1% à 50% en poids de la composition.

13. Composition pour une utilisation selon l'une quelconque des revendications 1-12, **caractérisée en ce que** chacun des acides est présent selon une quantité de 2-10% en poids de la composition totale.

14. Composition pour une utilisation selon l'une quelconque des revendications 1-13, **caractérisée en ce que** chaque acide quelconque ne constitue pas plus de 75% de la combinaison.

15. Composition pour une utilisation selon l'une quelconque des revendications 1-14, **caractérisée en ce que** la composition comprend en outre un agent d'augmentation de la viscosité.

16. Composition pour une utilisation selon la revendication 15, **caractérisée en ce que** l'agent d'augmentation de la viscosité est choisi dans le groupe constitué par les dérivés de cellulose solubles, l'hydroxyéthylcellulose, les oligosaccharides, les polysaccharides, le polyéthylène glycol, le polypropylène glycol ayant un poids moléculaire supérieur à 1000 grammes par mol, la silice pyrogénée et l'oxyde d'aluminium.

17. Composition pour une utilisation selon la revendication 16, **caractérisée en ce que** l'agent d'augmentation de la viscosité est l'hydroxyéthylcellulose ou la silice pyrogénée.

18. Composition pour une utilisation selon l'une quelconque des revendications 1-17, **caractérisée en ce que** l'infection fongique est une infection de la peau ou des ongles.

19. Composition pour une utilisation selon l'une quelconque des revendications 1-18, **caractérisée en ce que** l'infection fongique est choisie dans le groupe constitué par *Tinea unguium, Tinea pedis, Tinea cruris, Tinea corporis, Tinea versicolor* et *Tinea candidiasis.*

20. Composition pour une utilisation selon l'une quelconque des revendications 1-16, **caractérisée en ce que** l'infection fongique est provoquée par *Trichophyton rubrum* ou *Trichophyton interdigitale.*

21. Préparation antifongique topique destinée au traitement d'infections fongiques, comprenant une combinaison d'acide propionique, d'acide formique et d'acide benzoïque, des sels de ceux-ci ou des dérivés d'anhydride ou d'ester de ceux-ci, solubilisés dans un véhicule hygroscopique non volatil, **caractérisée en ce que** la préparation comprend lesdits acides selon une quantité allant de 0,1% à 50% en poids de la composition, et aucun acide ou sel seul ne constitue plus de 75% en poids de la teneur en acide totale.

22. Préparation selon la revendication 21, **caractérisée en ce que** les acides sont sous forme de sel.

23. Préparation selon la revendication 22, **caractérisée en ce que** le sel est un sel métallique.

24. Préparation selon la revendication 23, **caractérisée en ce que** le métal est choisi dans le groupe constitué par le sodium, le potassium, le calcium et le zinc.

25. Préparation selon l'une quelconque des revendications 21-24, **caractérisée en ce que** la composition comprend une combinaison de formiate de sodium, de propionate de zinc, de propionate de calcium et de benzoate de sodium.

26. Préparation selon l'une quelconque des revendications 21-25, **caractérisée en ce que** le véhicule hygroscopique non volatil est choisi dans le groupe constitué par les solvants polyhydroxylés, et les polyglycols ayant des poids moléculaires inférieurs à 500 grammes par mol.

27. Préparation selon l'une quelconque des revendications 21-26, **caractérisée en ce que** le véhicule hygroscopique non volatil est choisi dans le groupe constitué par le glycérol, le diglycérol, l'éthylène glycol, le propylène glycol, le sorbitol, le xylitol, le maltitol, le polyéthylène glycol et le polypropylène glycol, ayant un poids moléculaire inférieur à 500 grammes par mol.

28. Préparation selon l'une quelconque des revendications 21-25, **caractérisée en ce que** le véhicule hygroscopique non volatil est choisi dans le groupe constitué par le propylène glycol et le glycérol.

29. Préparation selon l'une quelconque des revendications 21-25, **caractérisée en ce que** le véhicule hygroscopique non volatil comprend du propylène glycol et du glycérol.

30. Préparation selon l'une quelconque des revendications 21-25, **caractérisée en ce que** le véhicule hygroscopique non volatil comprend du propylène glycol et de l'hydroxyéthylcellulose.

31. Préparation selon l'une quelconque des revendications 21-30, **caractérisée en ce que** chacun des acides est présent selon une quantité de 2-10% en poids de la composition totale.

32. Préparation selon l'une quelconque des revendications 21-31, **caractérisée en ce que** la préparation comprend en outre un agent d'augmentation de la viscosité.

33. Préparation selon la revendication 32, **caractérisée en ce que** l'agent d'augmentation de la viscosité est choisi dans le groupe constitué par les dérivés de cellulose solubles, l'hydroxyéthylcellulose, les oligosaccharides, les polysaccharides, le polyéthylène glycol, le polypropylène glycol ayant un poids moléculaire supérieur à 1000 grammes par mol, la silice pyrogénée et l'oxyde d'aluminium.

34. Préparation selon la revendication 33, **caractérisée en ce que** l'agent d'augmentation de la viscosité est l'hydroxyéthylcellulose ou la silice pyrogénée.

35. Bandage ou autre matériau à appliquer sur de la peau ou des ongles infectés par un champignon, imprégné d'une préparation selon l'une quelconque des revendications 21-34.
